# EUROPEAN PATENT SPECIFICATION

(11) **EP 0 360 458 B1**
(45) Date of publication and mention of the grant of the patent: **11.08.1993**
(21) Application number: 89309035.7
(22) Date of filing: 06.09.1989
(51) Int. Cl.: A61F 13/02

(54) **Adhesive dressing, its preparation and use**
Klebverbände, ihre Herstellung und Anwendung
Bandage adhésif, sa préparation et son utilisation

(30) Priority: 07.09.1988 GB 8820944; 14.12.1988 GB 8829151; 02.05.1989 US 345987
(43) Date of publication of application: 28.03.1990
(73) Proprietor: SMITH & NEPHEW plc, London WC2R 3BP (GB)
(72) Inventor: Ward, William John, Hull HU5 3JP (GB)
(74) Representative: Gilholm, Stephen Philip

(56) References cited:
- EP-A- 0 243 069
- EP-A- 0 284 219
- WO-A-88/02248
- US-A- 4 646 731

## Description

The present invention relates to an adhesive dressing which comprises a backing layer which has on one surface a pressure sensitive adhesive layer covered by a removable release liner and attached to the other surface a support layer. The adhesive dressing may be used to treat wound such as burns, abrasions, ulcers and surgical incisions and as a protective dressing to cover for example an indwelling catheter site.

One type of adhesive dressing which has been used for many years comprises a thin, filmic, moisture vapour permeable polymeric material coated on one surface with an adhesive. The dressing was presented for use with a protector layer over the adhesive and adhesive-free handles at a pair of opposed edges. Subsequently alternative modes of presentation were introduced to ease application of the thin filmic product for the inexperienced particularly in an effort to prevent the dressing creasing and adhering to itself during application. If this occurs then the dressing must be discarded. One such mode of presentation required the presence of a carrier means usually of a material stiffer than the adhesive coated film adhered to the non-adhesive surface of that film. To apply such dressings the release liner was removed, the adhesive coated film was adhered to the patient and then the carrier removed. Dressings of this type are described in for example GB-A-2120104, EP-A-51935, EP-A-66899, EP-A-81990, US-A-4372303, US-A-4374520, US-A-4513739, US-A-455371 and US-A-4600001.

WO-A-88/02248 describes a package assembly for a plastic film bandage inter alia comprising an adhesive plastic film bandage and a tube anchored to the adhesive side of the bandage along an edge. A support sheet is removeably attached to the non-adhesive surface of the plastic film bandage and extends beyond the edge of the film bandage. A peel-away adhesive protector means extends over both the adhesive surface of the film bandage and the tube and extends beyond an edge of the bandage and is attached to the support by hinge runs whereby upon removal of the protector means from the adhesive surface it serves as a grippable extension of the support sheet.

In US-A-4646731 there is disclosed an adhesive bandage having an adhesive protective member covering end portions and which is supported on a removeable protective backing sheet which covers other parts covering the end portion is folded into a V-shaped strip having two superposed layers the ends of which extend beyond the dressing. In use the bandage, for example, a suture in removed from the supporting backing sheet thus exposing a central area of adhesive. The bandage is then applied, e.g. over an incision, followed by the removal of the folded tab strip from the adhesive ends of the suture.

A simple way of presenting thin film dressings for application to a patient has been found which avoids the complexity of previously known dressings, which avoids the requirement of tearing or cutting any part of the dressing during application except when fitting to an awkward body part such as the heel but even then as with the normal dressing the presence of the support layer reduces the risk of the dressing adhering to itself or being contaminated by accidental contact with non-sterile surfaces. If the dressing has to be cut as when fitting an awkward body part such as the heel, then the presence of the support layer over the whole of the remainder of the dressing facilitates its application. this is in contrast to, for example, those dressings which use devices such as handles or frames to support as this support may be lost if these devices are cut through. The dressings of the present invention may be readily applied using only one hand.

The support layer is conformable by which it is meant that the support layer will conform to the contours of a surface to which it is applied. Aptly the support layer may be left attached to the backing layer without detracting seriously from the performance of the dressing, though it is preferred to remove the support layer.

Suitably the removable protector and the support layer extend beyond the backing layer at one edge but more suitably the removable protector and support layer extend beyond the backing layer at two opposed edges.

The term 'opposed edges' also includes opposed edge regions, in these instances, such as with circular or oval dressings where the limits of each separate edge may not be readily apparent and the edges may merge.

The protector and support layers may extend peripherally beyond the backing layer.

Suitably the backing layer may comprise any of those materials which are conventionally employed to form thin film surgical dressing. Suitable materials include those described in United Kingdom Patent No. 1280631, European Patents Nos. 51935, 91800 and 178740. Particularly apt materials are polyurethanes, for example polyester or polyether polyurethanes known as Estanes (Trade Mark). Other apt materials are elastomeric polyether polyesters, for example those known as Hytrels (Trade Mark) and polyether polyamides, for example those known as Pebaxes (Trade Mark). Other favoured materials include hydrophilic polymers such as hydrophilic polyurethanes including those described in United Kingdom Patent No. 2093190B, especially the polyurethane described in Example 2 therein. Such materials will typically take up from 5 to 95% by weight of water.

The materials employed in the dressings of the invention may be moisture vapour permeable.

The moisture vapour transmission rate of the materials employed in the present invention may be measured by a procedure known as the Payne Cup method. The method uses a cup 1.5cm deep with a flanged top. The inner diameter of the flange is such to provide an area for moisture vapour transmission of 10cm². In this method 10ml of distilled water is added to the cup and a sample of the material under test, large enough to completely cover the flange, is clamped over the cup. The complete assembly is then weighed and placed in a cabinet where the temperature and relative humidity are maintained at 37°C and 10% respectively. After 17 hours the cup is removed from the cabinet and allowed to cool at room temperature. After re-weighing, the mass of water lost by vapour transmission is calculated and the result expressed as in g/m²/24hrs at 37°C at 100% to 10% relative humidity difference. Hereinafter the units for moisture vapour transmission will be abbreviated to gm⁻².

Suitably the backing layer is moisture vapour permeable and has a moisture vapour transmission rate of at least 500gm⁻² relative humidity difference, more suitably at least 1200gm⁻² and preferably at least 1600gm⁻².

Suitably the backing layer has thickness of from 15 to 100µm, more suitably 20 to 80µm and preferably 25 to 50µm, for example 27.5µ, 30µm, 35µm and 40µm.

Aptly the pressure sensitive adhesive layer may be formed from an adhesive which is conventionally used for contact with the skin. Suitable adhesives include polyvinyl alkyl ether adhesive and acrylate ester copolymer adhesives. Suitable adhesives are described in GB-A-1280631, EP-A-35399 and EP-A-51935. Preferably the adhesive is a polyvinyl ether adhesive or an acrylate ester copolymer adhesive formed by the copolymerisation of 2-ethylhexyl acrylate, butyl acrylate and acrylic acid.

Suitably the adhesive layer is from 15 to 65µm thick, for example 20 to 40µm thick and is applied at a weight per unit area of 10 to 75gm⁻², more suitably 15 to 65gm⁻² and preferably 25 to 40gm⁻².

Suitably the removable protector is a silicone coated release paper. Suitably the removable protector may have a weight per unit area of 100 to 140 g m⁻², and preferably 110 to 130g m⁻², for example 120g m⁻². The removable protector may be divided into two or more pieces. Preferably at least one of the protector pieces is significantly larger than the other or others and covers a major proportion of the adhesive layer. It is desirable that the stripping load of the support layer from the backing layer is greater than that of the protector from the adhesive layer otherwise there is a risk that the support layer would peel from the backing layer before the protector can be removed.

The removable protector may extend less beyond the edge of the backing layer than the corresponding support layer. This facilitates grasping the support layer in one hand and the protector in the other.

The smaller piece has a portion extending away from the adhesive surface and is folded back to form a V-shape with the fold line or apex of the V being away from the edge of the dressing is towards the interior of the dressing. The larger piece of the removable protector then covers the remaining adhesive surface and overlaps onto the V-shaped piece.

Preferably at least one, more preferably, both edges of the V-shaped part extends beyond the edge of the backing layer. The edge of the overlying portion of the second part also extends beyond the edge of the backing layer.

In use the support layer and the V-shaped piece are gripped in one hand and the second part of the adhesive protector peeled off with the other. Alternatively only the V-shaped piece may be gripped. When the major portion of the dressing is in place the V-shaped piece may then be removed and the remainder of the dressing applied to the patient. Then if desired the support layer is removed. Alternatively, if desired, the conformability of the dressing may be increased by removing the V-shaped piece first and the dressing handled aseptically by the projecting edges of the support layer.

Suitably the support layer is formed from a polymeric film or a paper. The support layer may be attached to the backing layer by virtue of casting or extruding the backing layer onto the support layer thereby forming an attachment which is easily reversed. Suitably the support layer may be formed from, for example, a transparent polymeric film such as a polyethylene or polypropylene film or from an opaque silicone or polyethylene coated paper. The support layer may carry markings including those in the form of a grid or concentric circles and the like whereby the progress of a healing wound or ulcer may be monitored. In this case the support layer remains on the backing layer.

The dressings of the present invention possess advantages over these of the prior art in that the dressing can be readily handled with one hand, the major portion of the adhesive surface of the dressing can be readily presented without creasing the dressing and yet the dressing is securely and aseptically held and, once applied, the remaining supporting and protecting components of the dressing can be easily removed without disturbing the portion of the dressing applied.

Suitably the adhesive dressing has a backing layer and adhesive layer which are translucent and preferably are transparent.

In a preferred embodiment of the invention, the support layer, backing and adhesive layer, constituting the dressing to be applied, are each sufficiently transparent such that the wound or catheter site can be viewed through the dressing once one or more of the adhesive protectors have been removed. The provision of transparent support layers together with other transparent dressing components not only enables the dressing to be both readily handled during application, usually with use of only one hand, but also enables the location of the dressing to be visible and for the dressing to be accurately positioned at the desired location.

The adhesive dressing may be prepared by casting a solution of the polymer which is to form the backing layer onto a long strip of the film which is to form the support layer. An adhesive may be cast or transfer coated onto the backing layer. The backing layer and adhesive layer may then be trimmed to the correct width on the support layer. The removable protector may then be applied to the adhesive surface in one or two pieces as described hereinbefore. The material so formed may be further trimmed and then cut transversely to form dressings of the appropriate size. The dressings may have an area equivalent to 5 x 5cm to 20 x 20cm, for example 5 x 7.5cm, 7.5 x 10cm, 10 x 14cm.

The adhesive dressing may be placed in a bacteria-proof pack, sealed and sterilised by conventional methods including using ethylene oxide or γ-irradiation.

In use the sterile adhesive dressing is removed from the pack, the removable protector is removed, the adhesive layer is applied to the skin of the patient and the support layer may then be removed.

Preferred embodiments of adhesive dressings of the present invention will now be described by way of example only and with reference to the drawings in which -
Figure 1 is a cross-section through one form of a dressing according to the invention.
Figure 2 is a cross-section of a dressing similar to that shown in Figure 1 but in which only the right-hand edge of the backing and adhesive layers is trimmed.
Figure 3 is a cross-section of a dressing similar to that shown in Figure 1 but in which the left-hand edge of the backing layer and adhesive layer is trimmed.

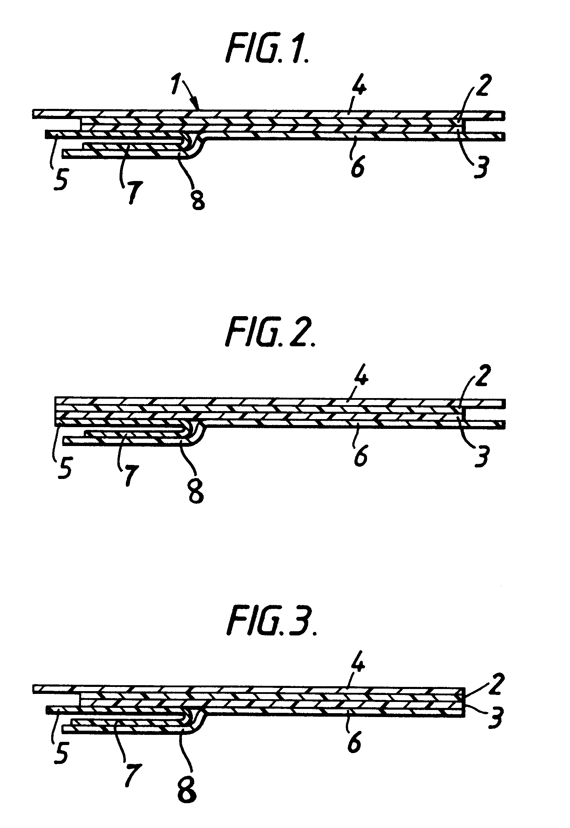

Figure 1, shows an adhesive dressing (1) which comprises a backing layer (2) formed from a film of a polyether polyurethane. The film has a weight per unit area of 30gm⁻² and a thickness of 27.5µm. The backing layer (2) has on one surface a pressure sensitive adhesive layer (3) formed from polyacrylate ester adhesive. The adhesive layer (3) has a weight per unit area of 30gm⁻². On the non-adhesive surface of the backing layer (2) is a support layer (4). The support layer (4) may comprise a silicone or polyethylene coated paper or a transparent film of polyethylene or polypropylene. A transparent or translucent support layer (4) may also carry markings for example in the form of a grid whereby the progress of healing wound or ulcer may be measured. The adhesive layer (3) is covered by removable protectors (5,6) made from a silicone coated release paper. The removable protectors (5,6) and support layer (4) extend beyond the backing layer (2). The smaller piece (5) is folded into a V-shape. The larger piece (6) is essentially flat and overlaps onto the smaller piece (5).

The length of the adhesive part of a typical small wound or IV dressing is 10cm and its width 12.0cm. The dressing may be packaged in a sealed peelable pouch and sterilised by ethylene oxide gas.

In use the larger piece (6) is removed first and the dressing held by the overlying portion of piece (5) and edge of the support layer (4). In such a case the larger area of the dressing is adhered to the skin, then the smaller piece (5) and the support layer (4) may be removed. Alternatively, the smaller protector piece (5) may be removed before application and the dressing handled aseptically by the edges of the support layer (4) which project beyond the adhesive (3) and film (2) layers.

Figures 2 and 3 show a dressing similar to that shown in Figure 1 wherein the edges of the dressings have been trimmed on the left-hand and right-hand sides respectively. The support layer (4) and the protector (5,6) extend beyond the adhesive and backing layers on the same one edge only.

## Claims

1. An adhesive dressing (1) which comprises a backing layer (2) having a pressure sensitive adhesive layer (3) over one surface thereof, a removable protector which covers the adhesive layer (3) and extends beyond the backing layer (2) at one or more edges and a conformable support layer (4) which is reversibly attached to the non-adhesive surface of the backing layer (2) and extends beyond the backing layer (2) at one or more or said edges, the protector comprises first part (5, 7) covering minor proportion of the adhesive layer and a second part (6, 8) covering a major proportion of the adhesive layer, characterized in that the first part (5) having a portion (7) extending away from the adhesive surface and bent back to form a V-shape and the second part (6) having a portion (8) which extends away from the adhesive surface to overlap and to extend beyond said portion (7) of the V-shaped first part (5).

2. A dressing as claimed in claim 1 wherein the support layer (4) is a transparent sheet material.

3. A dressing as claimed in any one of the preceding claims wherein at least one of the ends of the first part (5,7) of the protection sheet extends beyond the edge of the backing layer (2).

4. A dressing as claimed in claim 3 wherein both ends of the first part (5,7) of the protector extends beyond the edge of the backing layer (2).

5. A dressing as claimed in any one of the preceding claims wherein the overlapping portion of the second part (6,8) of the protector extends beyond the edge of the backing layer.

6. A dressing as claimed in any one of the preceding claims wherein the protector extends beyond opposed edges of the backing layer (2).

7. A dressing as claimed in any one of the preceding claims wherein the support layer (4) extends beyond the edge of the backing layer (2) further than the protector.

8. A dressing as claimed in any one of the preceding claims in which the backing layer (2) is moisture vapour permeable.

9. A dressing as claimed in claim 8 wherein the backing layer (2) has moisture vapour transmission rate of at least 1200gm⁻².

10. A dressing as claimed in any one of the preceding claims wherein the backing layer is a film of polyester or polyether polyurethane, an elastomeric polyether polyester or polyether polyamide.

11. A dressing as claimed in any one of the preceding claims in which the backing layer is a sheet of hydrophilic polyurethane.

12. A dressing as claimed in any one of the preceding claims wherein the backing layer is formed from a polymeric film or coated paper.

13. A dressing as claimed in claim 12 wherein the polymeric film is a polyethylene or polypropylene film.

14. A dressing as claimed in any one of the preceding claims, in a sterilised form and sealed within a bacteria-proof pack.

## Patentansprüche

1. Klebeverband (1) mit einer Trägerschicht (2), die eine druckempfindliche Klebeschicht (3) an einer Oberfläche aufweist, einer abnehmbaren Schutzlage, die die Klebeschicht (3) bedeckt und sich an einer oder mehreren Kanten über die Trägerschicht (2) hinaus erstreckt, und einer anpaßbaren Stützschicht (4), die lösbar an der nicht-klebenden Oberfläche der Trägerschicht (2) angebracht ist und sich über die Trägerschicht (2) an einer oder mehreren der genannten Kanten hinaus erstreckt, wobei die Schutzlage einen erstem Teil (5, 7),der einen kleineren Bereich der Klebeschicht bedeckt, und einen zweiten Teil (6, 8) aufweist, der einen größeren Bereich der Klebeschicht bedeckt, dadurch gekennzeichnet, daß der erste Teil (5) einen Teilbereich (7) aufweist, der sich von der Klebe-Oberfläche weg erstreckt und zurückgebogen eine V-Form annimmt, und der zweite Teil (6) einen Teilbereich (8) aufweist, der sich von der Klebe-Oberfläche weg erstreckt und den genannten Bereich (7) des V-förmigen ersten Teils (5) überlappt und sich über diesen hinaus erstreckt.

2. Verband nach Anspruch 1, bei dem die Stützschicht (4) ein transparentes Folienmaterial ist.

3. Verband nach einem der vorhergehenden Ansprüche, bei dem sich wenigstens eines der Enden des ersten Teils (5, 7) der Schutzfolie über die Kante der Trägerschicht (2) hinaus erstreckt.

4. Verband nach Anspruch 3, bei dem sich beide Enden des ersten Teils (5, 7) der Schutzlage über die Kante der Trägerschicht (2) hinaus erstreckt.

5. Verband nach einem der vorhergehenden Ansprüche, bei dem sich der überlappende Bereich des zweiten Teils (6, 8) der Schutzlage über die Kanten der Trägerschicht hinaus erstreckt.

6. Verband nach einem der vorhergehenden Ansprüche, bei dem die Schutzlage sich über gegenüberliegende Kanten der Trägerschicht (2) hinaus erstreckt.

7. Verband nach einem der vorhergehenden Ansprüche, bei dem die Stützschicht (4) sich über die Kante der Trägerschicht (2) weiter hinaus erstreckt als die Schutzlage.

8. Verband nach einem der vorhergehenden Ansprüche, bei dem die Trägerschicht durchlässig für Wasserdampf ist.

9. Verband nach Anspruch 8, bei dem die Trägerschicht (2) eine Durchlässigkeit für Wasserdampf von wenigstens 1200 gm⁻² hat.

10. Verband nach einem der vorhergehenden Ansprüche, bei dem die Trägerschicht ein Film aus Polyester- oder Polyetherpolyurethan, ein elastomeres Polyetherpolyester oder ein Polyetherpolyamid ist.

11. Verband nach einem der vorhergehenden Ansprüche, bei dem die Trägerschicht ein Film aus hydrophilem Polyurethan ist.

12. Verband nach einem der vorhergehenden Ansprüche, bei dem die Trägerschicht aus einem Polymerfilm oder einem beschichteten Papier gebildet ist.

13. Verband nach Anspruch 12, bei dem der Polymerfilm ein Polyethylen- oder ein Polypropylenfilm ist.

14. Verband nach einem der vorhergehenden Ansprüche in sterilisierter Form in einer bakteriendichten Verpackung abgeschlossen.

## Revendications

1. Pansement adhésif (1) qui comprend une couche de base (2) comportant une couche d'adhésif sensible à la pression (3) sur une de ses surfaces, un protecteur détachable qui recouvre la couche adhésive (3) et s'étend au-delà de la couche de base (2) sur un ou plusieurs bords, et une couche support conformable (4) qui est attachée de façon réversible à la surface non adhésive de la couche de base (2) et s'étend au-delà de la couche de base (2) sur un ou plusieurs desdits bords, le protecteur comprenant une première partie (5,7) qui couvre une proportion mineure de la couche adhésive et une deuxième partie (6,8) qui couvre une proportion majeure de la couche adhésive, caractérisé en ce que la première partie (5) comprend une portion (7) détachée de la surface adhésive et rabattue de manière à définir une forme en V, et la deuxième partie (6) comprend une portion (8) qui s'écarte de la surface adhésive de manière à chevaucher ladite portion (7) de la première partie en forme de V (5) et à s'étendre au-delà de cette portion (7).

2. Pansement suivant la revendication 1, dans lequel la couche support (4) est une feuille de matière transparente.

3. Pansement suivant l'une quelconque des revendications précédentes, dans lequel au moins une des extrémités de la première partie (5,7) de la feuille de protection s'étend au-delà du bord de la couche de base (2).

4. Pansement suivant la revendication 3, dans lequel les deux extrémités de la première partie (5, 7) du protecteur s'étendent au-delà du bord de la couche de base (2).

5. Pansement suivant l'une quelconque des revendications précédentes, dans lequel la portion chevauchante de la deuxième partie (6,8) du protecteur s'étend au-delà du bord de la couche de base.

6. Pansement suivant l'une quelconque des revendications précédentes, dans lequel le protecteur s'étend au-delà des bords opposés de la couche de base (2).

7. Pansement suivant l'une queconque des revendications précédentes, dans lequel la couche support (4) s'étend au-delà du bord de la couche de base (2), plus loin que le protecteur.

8. Pansement suivant l'une quelconque des revendications précédentes, dans lequel la couche de base (2) est perméable à la vapeur d'eau.

9. Pansement suivant la revendication 8, dans lequel la couche de base (2) a une capacité de transmission de vapeur d'eau d'au moins 1200 g/m².

10. Pansement suivant l'une quelconque des revendications précédentes, dans lequel la couche de base est un film de polyester ou de polyéther polyuréthane, ou d'un polyéther polyester ou polyéther polyamide élastomère.

11. Pansement suivant l'une quelconque des revendications précédentes, dans lequel la couche de base est une feuille de polyuréthane hydrophile.

12. Pansement suivant l'une quelconque des revendications précédentes, dans lequel la couche de base est formée à partir d'un film de polymère ou d'un papier revêtu.

13. Pansement suivant la revendication 12, dans lequel le film de polymère est un film de polyéthylène ou de polypropylène.

14. Pansement suivant l'une quelconque des revendications précédentes, sous forme stérilisée et contenu de façon hermétique à l'intérieur d'un emballage étanche aux bactéries.
